Europäisches Patentamt

European Patent Office

Office européen des brevets

(11) Publication number: **0 044 054**
**A1**

## (12) EUROPEAN PATENT APPLICATION

(21) Application number: **81105394.1**

(22) Date of filing: **10.07.81**

(51) Int. Cl.³: **A 61 N 1/20**

(30) Priority: **14.07.80 IT 2342880**

(43) Date of publication of application:
**20.01.82 Bulletin 82/3**

(84) Designated Contracting States:
**AT BE CH DE FR GB LI SE**

(71) Applicant: **Pinferetti, Marco**
**Via Rovello, 19**
**I-20100 Milano(IT)**

(71) Applicant: **Ricci, Aldo**
**Corso Pavia, 22**
**I-27029 Vigevano(IT)**

(72) Inventor: **Pinferetti, Marco**
**Via Rovello, 19**
**I-20100 Milano(IT)**

(72) Inventor: **Ricci, Aldo**
**Corso Pavia, 22**
**I-27029 Vigevano(IT)**

(72) Inventor: **Peruzzini, Vincenzo**
**Piazza Piemonte, 5**
**I-20077 Melegnano(IT)**

(74) Representative: **Modiano, Guido et al,**
**MODIANO & ASSOCIATI S.A.S. Via Meravigli, 16**
**I-20123 Milan(IT)**

(54) Device for facilitating the formation of bone callus in bone fractures.

㊄ A device effective to facilitate the formation of bone callus following a fracture and reduction, either with or without surgical intervention, thereof; the device being of the type adapted for surgical introduction into the body of a patient. The device comprises at least one DC voltage generator (2) adapted to be connected to the fractured bone and a silicon controlled rectifier switch (SCR) which is normally open and adapted to change over to the closed condition through the interaction of an activating element (M) located outside of the body, thereby the circuit is closed between the generator (2) and bone and a current of preset intensity is passed through the bone itself.

Fig. 1

EP 0 044 054 A1

Croydon Printing Company Ltd.

This invention relates to a device for facilitating the formation of the so-called "bone callus", or calcification, following a bone fracture and reduction thereof, either with or without surgical intervention.

As is known, to repair bone fractures, it is of fundamental importance that large amounts of calcium be delivered to the fractured region for the setting of its fragments and regeneration of the fractured bone, a basic component whereof happens to be calcium phosphate. In particular, it has been shown that the metabolism of calcium and the bone process of mineralization are directly controlled both by vitamin D and the flow of an electric current of suitable intensity through the bone, which current promotes the absorption of calcium by ion exchange, regulates the calcium-phosphor balance required for the mineralization of the bone, and in particular greatly accelerates the formation of the bone callus, and accordingly the recovery process.

In the orthopedic field, already known are devices intended for surgical introduction into the body in order to cause a direct current to flow through a fractured bone. Such conventional devices generally include a battery, to the terminals whereof the fractured bone is connected with the optional interposition of a current stabilizing network. The battery-to-bone connection is such that a preset current can be passed through the bone as soon as the device is introduced into the body. This continuous flow of current, as initiated with the introduction of the device, has, however, adverse effects upon the healing of the wound generally resulting from the fracture reduction surgery, so that while accelerating the bone callus formation time, the cited conventional devices are often annoying for the patient, who frequently refuses them.

Thus, it is a primary object of the invention to provide a device of the type mentioned in the preamble, which exhibits no such drawbacks as the cited one affecting similar conventional devices, and which in particular can be activated at the desired time without adversely affecting the healing of the wound open by the **fracture-reduction surgical intervention**   or by the introduction of the device itself.

Another object of the invention is to provide a device which is effective to **facilitate the formation** of the bone callus, and is circuit-wise extremely simple, functional, and quite safe for the patient.

It is a further object of this invention to provide a device as indicated, which has minimized overall dimensions, thereby it can be easily introduced into the body, and which has a very small cost.

These and other objects, such as will be apparent hereinafter, are achieved by a device for **facilitating** the formation of bone callus in bone fractures, of the type suitable for surgical introduction into the body, comprising at least one DC voltage generator having first and second terminals, at least two leads connected with one end to said terminals and adapted for connection, with the other end, to a fractured bone, characterized in that it includes, in at least one of said leads, normally open switch means opening the circuit between said generator and said bone and being adapted for controlled change over to the closed condition through the interaction of an activating means located externally to the body, thereby said circuit between said generator and bone is closed, thus causing a current of preset intensity to flow through said bone.

Further features and advantages of the device according to the invention will be more clearly apparent from the following detailed description of an embodiment thereof, as illustrated by way of example and not of limitation in the accompanying drawing., where:

Figure 1 shows a currently preferred embodiment of the device for **facilitating the formation of bone** callus according to the invention; and

Figure 2 shows one possible circuit diagram of a current **regulator** intended for association with the device of Figure 1 in order to stabilize the current supply therefrom.

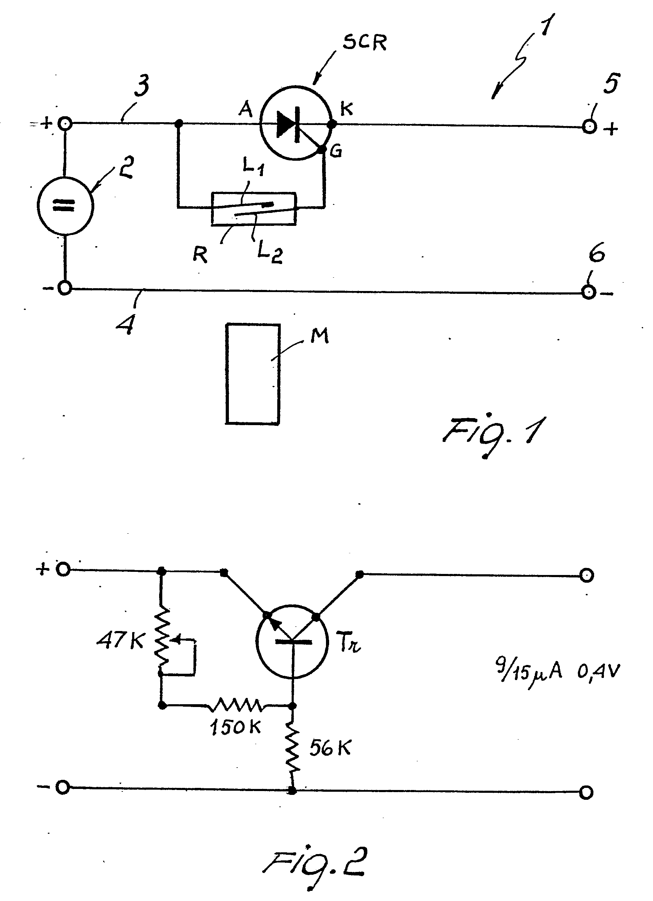

With specific reference to the drawing figures, and particularly to Figure 1, there is shown a general electric diagram of the inventive device, said device being generally indicated at 1. As may be seen, the device 1 includes a DC generator 2, e.g. in the form of a conventional miniaturized or button-type battery capable of supplying a few volts and having a plus terminal and a minus terminal, and of an SCR serially arranged in the lead 3 connected to the plus terminal, another lead 4 being connected to the minus terminal of the generator 2. More specifically, the anode of the SCR, as indicated at A, is connected to said plus terminal of the generator, the cathode K of the SCR being connected to a plus terminal 5, while the electrode of gate G of said SCR is connected through a protected magnetic laminations relay or reed relay R, to the plus terminal of the generator 2. A further minus terminal 6 is also provided for either direct connection to the bone, or in most cases, to a voltage regulating circuit which will be more specifically described hereinafter.

The reed relay R is intended for subcutaneous implantation in close proximity of the skin, such that its reeds $L_1$ and $L_2$ can be closed, for example, by passing a magnet M over the body in the vicinity of said reed relay R. In the situation shown in Figure 1, with the reeds $L_1$, $L_2$ of the reed relay R being open, no current can flow between the terminals 5 and 6, even with the load (bone) connected. In fact, the SCR is selected to be, with the reeds $L_1$ and $L_2$ in the open condition, in a direct blocking state, the changing over of the SCR to the forward conducting state being only allowed upon the occurrence of a gating pulse to be obtained, as those skilled in the art will readily recognize, by closing

the reeds $L_1$, $L_2$ under the effect of the magnet $M$, the gate of the SCR being in this case connected to the plus side of the generator 2. Obviously, once energized, the SCR will remain conductive practically until the battery has been exhausted.

In Figure 2, for completion sake, there is shown a general circuit diagram of a voltage regulator adapted for cascade connection between the terminals 5 and 6 to regulate or stabilize the voltage supplied by the generator 2. This voltage regulator, which comprises an NPN transistor $Tr$, will be no further described because well known to the expert. It will be sufficient to remark that, thanks to the base-connected trimmer, it becomes possible to regulate the current supplied within a fairly wide range, the regulation being of the parallel type and an optimum value being, as ascertained through actual tests, 0.4 Volts with the current in the 9 to 20 microAmp range.

Of course, the electronic device of this invention will be enclosed in a suitable sterile material container which will be compatible with the surgical requirements and in particular with the introduction into the body. The overall size of the circuit has been minimized, such that the device can cause no inconvenience to the patient.

It will be appreciated from the foregoing that the invention fully achieves its objects; in particular a device for facilitating the formation of bone callus has been provided which, contrary to similar conventional devices, does not feed the bone in a continuous manner, but only feeds it upon control from the outside, after the wound has healed. The device, moreover, is reliable in operation. because it is free of such critical factors as, for example, bias effects, transistor gain, oxidation of relay contacts, etc., which are all apt to jeopardize the attainment of good final results.

The invention as conceived is susceptible to many modifications and variations, without departing from the purview of the instant inventive concept. As an example, susceptible to modification is the particular circuit layout selected for activating the device at the desired time, the invention essence residing in the utilization of normally open switching means adapted to be closed at a desired time to stimulate the bone. In particular, instead of the reed relay, the switching element may comprise, for instance, a resonant circuit operative to energize the control electrode of the SCR through the interaction with a magnetic field of preset frequency, which is produced externally to the body. In addition to the reed relay, a suitable gating current limiting resistor may be connected in series during the turning on of the SCR.

Furthermore, while the invention has been described with specific reference to its application to the formation of bone callus, it should be noted that the instant device may also be used in all those applications where an apparatus introduced into the body is to be activated from the outside at a preset time, one typical case being that of a pace-maker.

Therefore, both the foregoing description and the accompanying illustrative drawing should be regarded as exemplary only, the invention spirit and scope being more clearly defined in the appended claims.

## CLAIMS

1. A device for facilitating the formation of bone callus in bone fractures, of the type suitable for surgical introduction into the body, comprising at least one DC voltage generator having first and second terminals, at least two leads connected with one end to said terminals and adapted for connection, with the other end, to a fractured bone, characterized in that it includes, in at least one (3) of said leads (3,4), normally open switch means (SCR) opening the circuit between said generator (2) and said bone and being adapted for controlled change over to the closed condition through the interaction of an activating means (M) located externally to the body, thereby said circuit between said generator and bone is closed, thus causing a current of preset intensity to flow through said bone.

2. A device according to Claim 1, characterized in that said normally open switch means comprise a silicon controlled rectifier (SCR) serially included in one (3) of said leads from said generator (2), said silicon controlled rectifier being normally in the off or forward blocking state thereof, the control or gating electrode (G) of said silicon controlled rectifier being connected, with the interposition of normally open relay means (R) to a potential of energization for said silicon controlled rectifier.

3. A device according to Claim 2, characterized in that said normally open relay means (R) comprise a protected magnetic laminations or reed relay ($L_1$, $L_2$), said reed relay being adapted to change over to the closed condition through the interaction of a magnet element (M) acting externally to the body.

4. A device according to Claim 2, characterized in that said energizing potential is the potential which appear on said (positive) lead.

5. A device according to Claim 1, characterized in that it includes, located downstream of said normally open switch means (SCR), transistorized voltage regulating means (Tr) operative to supply a regulated voltage to said bone at current levels which can vary within a preset range.

6. A device for facilitating the formation of bone callus in bone fractures, of the type suitable for surgical introduction into the body comprising, in a non-reactive sterile material container and substantially sealed tight therein:

a) a DC voltage generator (2) having a first (positive) terminal and second (negative) terminal;

b) two leads connected, with one end, to said (positive, negative) terminals of said generator and being adapted for connection, with the other end and through optional probes, to the fractured bone,

characterized in that it further comprises:

c) a normally open solid state switch (SCR) serially included in said (positive) lead, said SCR being normally in the off or forward blocking condition, the control or gating electrode (G) of said SCR being connected, with the interposition of a normally open reed relay (R), to said (positive) lead, said reed relay (R) being adapted for change over to the closed condition through the interaction of a magnet element (M) acting externally to the body, thereby said SCR is substantially turned on by application of said potential appearing on said (positive) lead to said control electrode (G) thereof; and

d) a transistorized voltage regulator (Tr) circuitally connected downstream of said SCR and adapted to stabilize the voltage such as to supply a stabilized voltage at currents which may vary within a wide range, the output terminals of said transistorized voltage regulator being the terminals whereto the load or fractured bone is applied.

7. A medical apparatus activable by means of a device according to any of the preceding claims.

Fig. 1

47K

150K

56K

$^9/_{15}\mu A$  0,4V

Fig. 2

0044054

European Patent Office

**EUROPEAN SEARCH REPORT**

Application number

EP 81 10 5394

| Category | Citation of document with indication, where appropriate, of relevant passages | Relevant to claim |
|---|---|---|
| | **DE - A - 2 552 523** (SYBRON)<br>* Page 3, paragraph 3; page 5, paragraph 1 * | 1,5,6 |
| | **FR - A - 1 440 902** (YISSUM)<br>* Page 1, left-hand column, paragraph 3; page 2, left-hand column, paragraph 3 * | 1,3,6 |
| | **US - A - 3 648 707** (MEDTRONIC)<br>* Column 11, lines 7-25; figure 5 * | 1-3 |
| | **FR - A - 1 371 162** (COTELEC)<br>* Page 2, left-hand column, paragraphs 3-6 and right-hand column, paragraph 3 * | 1-4 |
| | **US - A - 3 311 111** (BOWERS)<br>* Column 2, lines 10-15; column 6, lines 58-66 * | 1,3,6 |
| | **US - A - 4 063 111** (DOBLER)<br>* Column 12, lines 59-69; figure 19 * | 2,4,6 |
| PE | **GB - A - 2 053 687** (GREATBATCH)<br>* Page 2, lines 14-34 and 110-126 *<br>& DE - A - 3 020 200<br>& FR - A - 2 461 506 | 1,3,6 |

**CLASSIFICATION OF THE APPLICATION (Int Cl.3)**

A 61 N    1/20

**TECHNICAL FIELDS SEARCHED (Int. Cl.3)**

A 61 N  1/36
        1/20
        1/32
        1/08

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 21-10-1981 | SIMON |

EPO Form 1503.1   06.78